# EUROPEAN PATENT APPLICATION

(11) **EP 4 085 973 A1**
(43) Date of publication of application: **09.11.2022**
(21) Application number: 21172160.0
(22) Date of filing: 04.05.2021
(51) Int. Cl.: A61P 29/00, C07K 16/18, A61K 39/00

(54) **INHIBITION OF EOSINOPHIL EXTRACELLULAR TRAPS**

(71) Applicant: Citryll B.V., 5349 AB Oss (NL)
(72) Inventor: The designation of the inventor has not yet been filed
(74) Representative: J A Kemp LLP

(57) **Abstract**

The invention provides methods for the inhibition of Eosinophil Extracellular Trap (EET) formation. In particular, the invention provides antibodies or binding fragments thereof directed against citrulline-containing epitopes, for use in methods to inhibit or detect EET formation. The methods may be for the diagnosis, treatment or prevention of any disease or condition which includes an EET-associated pathology.

## Description

### Field of the Invention

The invention provides methods for the inhibition of Eosinophil Extracellular Trap (EET) formation. In particular, the invention provides antibodies or binding fragments thereof directed against citrulline-containing epitopes, for use in methods to inhibit or detect EET formation. The methods may be for the diagnosis, treatment or prevention of any disease or condition which includes an EET-associated pathology.

### Background of the invention

Eosinophils are a form of circulating leukocyte, typically representing about 1 to 3% of white blood cells (WBCs) in a healthy human. They have a wide variety of roles in homeostasis and various diseases including allergy and infection. It has been identified that a particular mechanism of active cytolytic eosinophil cell death releases eosinophil extracellular traps (EETs) and total cellular contents. This is referred to as eosinophil extracellular trap cell death (EETosis). It has also been shown that Charcot-Leyden crystals (a classical pathological marker of eosinophilic inflammation) is associated with EETosis, and the presence of EETosis and EETs has been reported in multiple diseases.

A superficially similar process characterised by the formation of neutrophil extracellular traps (NETs) has been identified in neutrophils, and is referred to as NETosis. NETs are associated with various pathologies and NET inhibition as a treatment is discussed in, for example, WO2009147201, WO2011070172, WO2016092082, and WO2020038963.

Although EETosis and NETosis have some characteristics in common, such as similar NADPH-oxidase dependent processes, and morphological changes in the nucleus and plasma/nuclear membrane rupture, there are also many differences. For example, neutrophils and eosinophils have differences in the structures of granules and the extracellular traps that form. When neutrophils undergo NETosis, the granules disintegrate intracellularly and thus granule proteins adhere to the NETs. By contrast, most of the granules in eosinophils are intact during the process of EETosis, resulting in the generation of free extracellular granules and granule protein-free EETs. Both NETs and EETs retain histones (i.e. chromatin structure), but EETs are thicker in diameter than NETs because of less extensive protease modification of chromatin. Histone citrullination (e.g. mediated by the enzyme PAD4) is known to play a key role in NET formation, but the evidence for a similarly significant role in EET formation is inconclusive.

Given these differences, further study into EETs/EETosis is required to give a detailed understanding of the roles played in homeostasis and disease pathogenesis, and into the mechanism of EET formation and how this may be inhibited. In other words, there remains a need for compounds for the treatment or prevention of EET-associated pathologies.

### Summary of the invention

There is conflicting evidence for the role of citrullination of histones in EET formation. Surprisingly the present inventors have found that an antibody that binds to citrullinated epitopes on the amino terminus of histones 2A and/or histone 4 is able to inhibit EETosis, and may thus be used in methods to inhibit EET formation. The methods may be for the treatment or prevention of an EET-associated pathology. Such pathologies may include: an eosinophilic disease of the skin; a respiratory eosinophilic disease; a gastro-intestinal eosinophilic disease; an allergic disease; or a helminth, fungal, viral, or bacterial infection.

Representative antibodies that bind to citrullinated epitopes on deiminated human histone 2A and histone 4 are described in for example, WO2009147201, WO2011070172, WO2016092082, and WO2020038963. Each of these documents, and the antibodies disclosed therein (including all CDR sequences, variable region sequences and constant region sequences of both the heavy and light chains), is herein incorporated by reference. In particular, the antibodies designated RhmAb2.102, RhmAb2.108, RhmAb2.109, RhmAb2.110, RhmAb2.111 RhmAb2.112, MQ22.101, MQ22.102 and MQ22.101b/d in WO2016092082, and any antigen binding fragment thereof, are each incorporated by reference. Similarly, the antibodies disclosed with identifiers of the format hMQ22.101x/y in WO2020038963, and any antigen binding fragment thereof, are each incorporated by reference. The antibodies disclosed in WO2020038963 are particularly preferred and are discussed in more detail below. The antibody referred to in WO2020038963 as hMQ22.101f/LC41 is most preferred. This antibody may be described herein as CIT-013. The present invention provides:
A method of inhibiting or detecting the formation of eosinophil extracellular traps (EETs), the method comprising administering an antibody or binding fragment thereof that specifically binds to a citrullinated epitope on deiminated human histone 2A and/or histone 4 to a sample or a subject. The sample or subject is preferably a sample or subject in which eosinophils are present.

The method may be for the prevention or treatment of a disease or condition in a subject, and thus may comprise administering said antibody or binding fragment thereof to the subject in a prophylactically or therapeutically effective amount.

The disease or condition which typically includes an EET-associated pathology. The disease or condition may be an eosinophilic disease or condition. Eosinophilic diseases and conditions may include: an eosinophilic disease or condition of the skin; a respiratory eosinophilic disease or condition; a gastro-intestinal eosinophilic disease or condition; an allergic disease or condition; or a helminth, fungal, viral, or bacterial infection.

Also provided is an antibody or binding fragment thereof that specifically binds to a citrullinated epitope on deiminated human histone 2A and/or histone 4 for use in the above methods, particularly the above methods for the prevention or treatment of a disease or condition in a subject. Also provided is an antibody or binding fragment thereof that specifically binds to a citrullinated epitope on deiminated human histone 2A and/or histone 4 for use in the manufacture of a medicament for use in the above methods for the prevention or treatment of a disease or condition in a subject.

The method may alternatively be for the *ex vivo* inhibition or detection of EET formation in a sample. The method may be used to diagnose the presence of an EET-associated pathology.

### Brief Description of the Sequence Listing

### Antibody nomenclature

CDR = complementarity-determining region.
VH = heavy chain variable domain.
VL = light chain variable domain.
CH = heavy chain constant domain.
CL = light chain constant domain.
msVH22.101 = mouse VH of therapeutic antibody.
msVL22.101 = mouse VL of therapeutic antibody.
hVH22.101x = humanized VH of therapeutic antibody, 'x' refers to the heavy chain.
hVL22.101y = humanized VL of therapeutic antibody, 'y' refers to the light chain.
hVH22.101(HC)x = optimized humanized VH of therapeutic antibody, '(HC)x' refers to the heavy chain.
hVL22.101(LC)y = optimized humanized VL of therapeutic antibody, '(LC)y' refers to the light chain.
hMQ22.101x/y = humanized therapeutic antibody, 'x' refers to the heavy chain, 'y' refers to the light chain.
hMQ22.101(HC)x/(LC)y = optimized humanized therapeutic antibody of the invention, '(HC)x' refers to the heavy chain, '(LC)y' refers to the light chain.

| **SEQ ID NO** | **Protein** | **Name** |
|---|---|---|
| 1 | protein | CDR1 of msVH22.101 and hVH22.101(HC)x |
| 2 | protein | CDR2 of msVH22.101 and hVH22.101 (HC)x |
| 3 | protein | CDR3 of msVH22.101 and hVH22.101 (HC)x |
| 4 | protein | CDR2 of msVL22.101 and hVL22.101(LC)y |
| 5 | protein | CDR3 of msVL22.101 and hVL22.101(LC)y |
| 6 | protein | CDR1 of hVL22.101LC17 |
| 7 | protein | CDR1 of hVL22.101LC21 |
| 8 | protein | CDR1 of hVL22.101LC27 |
| 9 | protein | CDR1 of hVL22.101LC41 |
| 10 | protein | CDR1 of hVL22.101LC42 |
| 11 | protein | hVH22.101f |
| 12 | protein | hVH22.101HC9 |
| 13 | protein | hVL22.101LC17 |
| 14 | protein | hVL22.101LC21 |
| 15 | protein | hVL22.101LC27 |
| 16 | protein | hVL22.101LC41 |
| 17 | protein | hVL22.101LC42 |
| 18 | protein | SEQ ID NO 1 from WO2016092082-Example 1, histone 2A |
| 19 | protein | SEQ ID NO 2 from WO2016092082, histone 4 |
| 20 | protein | Shortened SEQ ID NO 2 from WO2016092082-Example 7, histone 4 |
| 21 | protein | Peptide no 4 (human histone 2A) (SEQ ID NO 24 from WO2011070172) |
| 22 | protein | Peptide no 6 (human histone 2A) (SEQ ID NO 26 from WO2011070172) |
| 23 | protein | Human heavy chain constant domain of IgG1 = preferred heavy chain constant domain of hCH22.101f |
| 24 | protein | Human kappa chain constant domain |
| 25 | protein | msVH22.101 |
| 26 | protein | hVH22.101j |
| 27 | protein | hVH22.101HC7 |
| 28 | protein | hVH22.101HC8 |
| 29 | protein | hVH22.101HC10 |
| 30 | protein | msVL22.101 |
| 31 | protein | hVL22.101e |
| 32 | protein | hVL22.101g |
| 33 | protein | hVL22.101h |
| 34 | protein | hVL22.101i |
| 35 | protein | hVL22.101i |
| 36 | protein | CDR1 of msVL22.101 and hVL22.101g |
| 37 | protein | CDR1 of hVL22.101e |
| 38 | protein | CDR1 of hVL22.101h |
| 39 | protein | CDR1 of hVL22.101i |
| 40 | protein | CDR1 of hVL22.101j |
| 41 | protein | CDR1 of hVL22.101LC16 |
| 42 | protein | CDR1 of hVL22.101LC19 |
| 43 | protein | CDR1 of hVL22.101LC20 |
| 44 | protein | CDR1 of hVL22.101LC22 |
| 45 | protein | CDR1 of hVL22.101LC23 |
| 46 | protein | CDR1 of hVL22.101LC24 |
| 47 | protein | CDR1 of hVL22.101LC25 |
| 48 | protein | CDR1 of hVL22.101LC26 |
| 49 | protein | CDR1 of hVL22.101LC37 |
| 50 | protein | CDR1 of hVL22.101LC38 |
| 51 | protein | CDR1 of hVL22.101LC39 |
| 52 | protein | CDR1 of hVL22.101LC40 |
| 53 | protein | msFibβ XG (SEQ ID NO 37 from WO2011070172) |
| 54 | protein | msVim XS/XL (SEQ ID NO 38 from WO2011070172) |
| 55 | Protein | Region around CDR2 of msVL22.101 and hVL22.101(LC)y |
| 56 | Protein | Alternative heavy chain constant domain of hCH22.101f-only difference relative to SEQ ID NO: 23 is an extra C terminal K, which is typically removed by intracellular processing. |

### Brief Description of the Figures

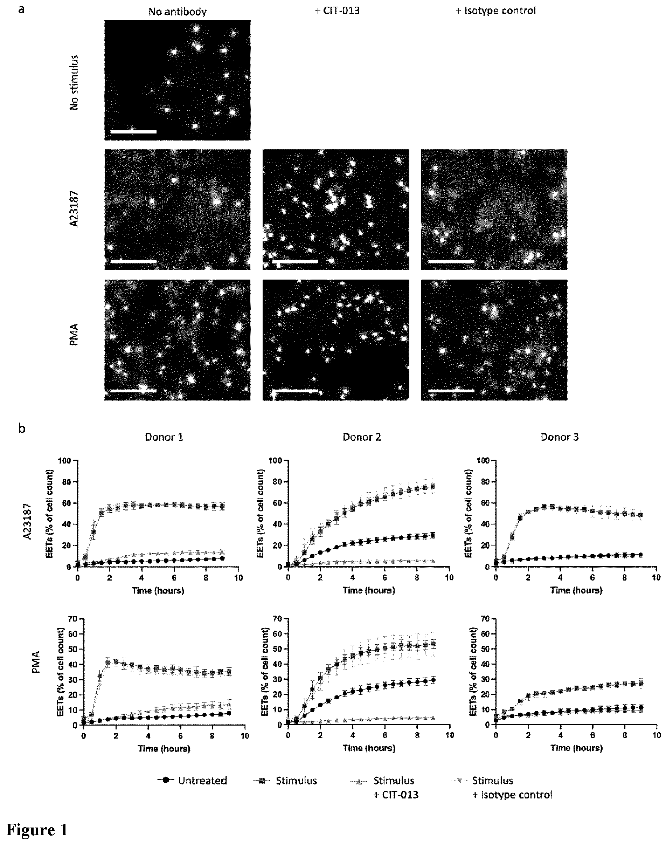

**Figure 1****.** (a) Representative images of eosinophils stimulated to form EETs with A23187 or PMA, in the presence of no antibody, CIT-013, or isotype control; (b) Graphical representation of the level of EET formation in samples of eosinophils from different donors, when stimulated under the same conditions as in panel (a).

### Detailed Description of the Invention

It is to be understood that different applications of the disclosed invention may be tailored to the specific needs in the art. It is also to be understood that the terminology used herein is for the purpose of describing particular embodiments of the invention only, and is not intended to be limiting.

In addition as used in this specification and the appended claims, the singular forms "a", "an", and "the" include plural references unless the content clearly dictates otherwise. Thus, for example, reference to "an antibody" includes "antibodies", and the like.

All publications, patents and patent applications cited herein, whether supra or infra, are hereby incorporated by reference in their entirety.

### Targets of an antibody or binding fragment thereof suitable for use in the methods of the invention

Citrulline is an amino acid that is not incorporated into proteins during normal translation, however, it may be generated by post-translational modification of an arginine residue by deiminating enzymes such as peptidyl arginine deiminase (PAD; EC 3.5.3.15). In mammals (humans, mice and rats), five PAD isotypes (PAD1 - PAD6; 'PAD4' and 'PAD5' are used for the same isotype), each encoded by a distinct gene, have been identified thus far. The terms demination and citrullination may thus be used interchangeably. Citrullination of human histone 2A and/or histone 4 may typically be carried out by, for example PAD2 and PAD4. Citrullination of these histones is associated with the pathological formation of NETs, but prior to the present invention there was no conclusive evidence that there is a comparable association with the pathological formation of EETs.

The antibodies or binding fragments thereof suitable for use in the methods of the invention specifically bind to a citrullinated epitope on deiminated human histone 2A and/or histone 4. The antibodies may also specifically bind to a citrullinated epitope on deiminated human histone H3. The antibodies or binding fragments thereof may specifically bind to a citrullinated epitope on deiminated human histone 2A and/or histone 4, wherein the epitope comprises a peptide selected from the group consisting of SEQ ID NOs: 18, 19, 20, 21 and 22. The antibodies or binding fragments thereof may also bind to epitopes comprising the peptides of SEQ ID NO: 53 or 54.

### Antibodies or binding fragments thereof

The term "antibodies", "antibody" or " binding fragment thereof" as used herein refers to a structure, preferably a protein or polypeptide structure, capable of specific binding to a target molecule often referred to as "antigen".

The antibody molecule as employed herein refers to an antibody or binding fragment thereof. The term 'antibody' as used herein generally relates to intact (whole) antibodies i.e. comprising the elements of two heavy chains and two light chains. The antibody may comprise further additional binding domains for example as per the molecule DVD-Ig as disclosed in WO 2007/024715, or the so-called (FabFv)₂Fc described in WO2011/030107. Thus 'antibody' as employed herein includes mono-, bi-, tri- or tetra-valent full-length antibodies.

Binding fragments of antibodies include single chain antibodies (i.e. a full-length heavy chain and light chain); Fab, modified Fab, Fab', modified Fab', F(ab')2, Fv, Fab-Fv, Fab-dsFv, single domain antibodies (e.g. VH or VL or VHH), scFv, mono-, bi-, tri- or tetra-valent antibodies, Bis-scFv, diabodies, tribodies, triabodies, tetrabodies and epitope-binding fragments of any of the above (see for example Holliger P and Hudson PJ, 2005, Nat. Biotechnol., 23,: 1126-1136; Adair JR and Lawson ADG, 2005, Drug Design Reviews - Online, 2, 209-217). The methods for creating and manufacturing these antibody fragments are well known in the art (see for example Verma R et al., 1998, J. Immunol. Methods, 216, 165-181). The Fab-Fv format was first disclosed in WO2009/040562 and the disulphide-stabilised versions thereof, the Fab-dsFv was first disclosed in WO2010/035012. Other antibody fragments for use in the present invention include Fab and Fab' fragments. Multi-valent antibodies may comprise multiple specificities e.g. bispecific or may be monospecific.

An antibody or binding fragment thereof may be selected from the group consisting of single chain antibodies, single chain variable fragments (scFvs), variable fragments (Fvs), fragment antigen-binding regions (Fabs), recombinant antibodies, monoclonal antibodies, fusion proteins comprising the antigen-binding domain of a native antibody or an aptamer, single-domain antibodies (sdAbs), also known as VHH antibodies, nanobodies (Camelid-derived single-domain antibodies), shark IgNAR-derived single-domain antibody fragments called VNAR, diabodies, triabodies, Anticalins, aptamers (DNA or RNA) and active components or fragments thereof.

IgG1 (e.g. IgG1/kappa) antibodies having an IgG1 heavy chain and a light chain may advantageously be used in the invention. However, other human antibody isotypes are also encompassed by the invention, including IgG2, IgG3, IgG4, IgM, IgA1, IgA2, IgAsec, IgD and IgE in combination with a kappa or lambda light chain. Also, all animal-derived antibodies of various isotypes can be used in the invention. The antibodies can be full-size antibodies or antigen-binding fragments of antibodies, including Fab, F(ab')2, single-chain Fv fragments, or single-domain VHH, VH or VL single domains.

The term: "specifically binds to citrulline" or "specifically binds to a citrullinated epitope" in this context means that the antibody or binding fragment thereof binds to a structure such as a peptide containing a citrulline residue whereas the antibody or binding fragment thereof binds less strongly or preferably not at all with the same structure containing an arginine residue instead of the citrulline residue. The term peptide should be interpreted as a structure that is capable of presenting the citrulline residue in the correct context for immunoreactivity with the antibodies or binding fragments thereof as described herein, preferably in the same context as it appears in the human or animal body, preferably in the context of a native polypeptide.

The antibodies or binding fragments thereof suitable for us in the methods of the invention specifically bind to a citrullinated epitope on deiminated human histone 2A and/or histone 4. The binding of antibodies or binding fragments thereof to a citrullinated epitope on deiminated human histone 2A and/or histone 4 blocks EET formation. Citrullination of histones is associated with the formation of EETs.

Blocking of EET formation can be total or partial. For example, the antibody or binding fragment thereof may reduce EET formation from 10 to 50%, at least 50% or at least 70%, 80%, 90%, 95% or 99%. EET blocking can be measured by any suitable means, for example by measuring EETosis *in vitro* (Fukuchi et al., "How to detect eosinophil ETosis (EETosis) and extracellular traps"; Allergology International, Volume 70, Issue 1, 2021, Pages 19-29).

The terms "binding activity" and "binding affinity" are intended to refer to the tendency of an antibody molecule to bind or not to bind to a target. Binding affinity may be quantified by determining the dissociation constant (Kd) for an antibody and its target. Similarly, the specificity of binding of an antibody to its target may be defined in terms of the comparative dissociation constants (Kd) of the antibody for its target as compared to the dissociation constant with respect to the antibody and another, non-target molecule.

Typically, the Kd for the antibody with respect to the target will be 2-fold, preferably 5-fold, more preferably 10-fold less than the Kd with respect to the other, non-target molecule such as unrelated material or accompanying material in the environment. More preferably, the Kd will be 50-fold less, even more preferably 100-fold less, and yet more preferably 200-fold less.

The value of this dissociation constant can be determined directly by well-known methods, and can be computed even for complex mixtures by methods such as those, for example, set forth in Caceci MS and Cacheris WP (1984, Byte, 9, 340-362). For example, the Kd may be established using a double-filter nitrocellulose filter binding assay such as that disclosed by Wong I and Lohman TM (1993, Proc. Natl. Acad. Sci. USA, 90, 5428-5432) or for example, by using Octet surface plasmon resonance.

One method for the evaluation of binding affinity for deiminated human histone 2A and/or histone 4 is by ELISA. Other standard assays to evaluate the binding ability of ligands such as antibodies towards targets are known in the art, including for example, Western blots, RIAs, and flow cytometry analysis. The binding kinetics (e.g. binding affinity) of the antibody also can be assessed by standard assays known in the art, such as surface plasmon resonance, for example by Biacore^{™} system analysis.

Preferably the antibody has a binding affinity for deiminated human histone 2A and/or histone 4 of 1 nM or less. Preferably the antibody of the invention has a binding affinity for deiminated human histone 2A and/or histone 4, and/or deiminated human histone H3 of 0.5 nM or less, 0.1 nM or less, 50 pM or less, 10 pM or less, 5 pM or less, 2 pM or less or 1 pM or less.

The antibody or binding fragment thereof may also be a fusion protein comprising the antigen-binding domain of a native antibody or an aptamer, such as an aptamer in the form of DNA or RNA.

Preferably the antibody is a monoclonal antibody. Monoclonal antibodies are immunoglobulin molecules that are identical to each other and have a single binding specificity and affinity for a particular epitope. Monoclonal antibodies (mAbs) of the present invention can be produced by a variety of techniques, including conventional monoclonal antibody methodology, for example those disclosed in "Monoclonal Antibodies: a manual of techniques"(Zola H, 1987, CRC Press) and in "Monoclonal Hybridoma Antibodies: techniques and applications" (Hurrell JGR, 1982 CRC Press).

The antibody or binding fragment thereof used in the methods of the invention comprises a binding domain. A binding domain will generally comprise 6 CDRs (3 in case of VHH), three from a heavy chain and three from a light chain. In one embodiment the CDRs are in a framework and together form a variable region or domain. Thus in one embodiment an antibody or binding fragment comprises a binding domain specific for the antigen comprising a light chain variable region or domain and a heavy chain variable region or domain.

The residues in antibody variable domains are conventionally numbered according to IMGT (http://www.imgt.org). This system is set forth in Lefranc MP (1997, J, Immunol. Today, 18, 509). This numbering system is used in the present specification except where otherwise indicated.

The IMGT residue designations do not always correspond directly with the linear numbering of the amino acid residues. The actual linear amino acid sequence may contain fewer or additional amino acids than in the strict IMGT numbering corresponding to a shortening of, or insertion into, a structural component, whether framework or CDR, of the basic variable domain structure. The correct IMGT numbering of residues may be determined for a given antibody by alignment of residues of homology in the sequence of the antibody with a "standard" IMGT numbered sequence.

The CDRs of the heavy chain variable domain are located at residues 27-38 (CDR1 of VH), residues 56-65 (CDR2 of VH) and residues 105-117 (CDR3 of VH) according to the IMGT numbering system.

The CDRs of the light chain variable domain are located at residues 27-38 (CDR1 of VL), residues 56-65 (CDR2 of VL) and residues 105-117 (CDR3 of VL) according to the IMGT numbering system.

Suitable antibodies or binding fragments thereof may be disclosed herein by the primary amino acid sequences of their heavy and light chain CDRs, their heavy and light chain variable regions, and/or their full length heavy and light chains.

A preferred antibody or binding fragment thereof for use in the methods of the invention comprises a modified VL CDR1 of an antibody or binding fragment thereof that specifically binds to a citrullinated epitope on deiminated human histone 2A and/or histone 4, which modified VL CDR1 provides improved properties to the antibody or binding fragment thereof over an antibody or binding fragment thereof comprising an unmodified version of CDR1 of the VL. The unmodified VL CDR1 comprises or consists of the amino acid sequences QSLLDSDGKTY (SEQ ID NO: 36) or QSLVDSDGKTY (SEQ ID NO: 37). Accordingly, an antibody or binding fragment thereof for use in the methods of the invention preferably does not include the VL CDR1 of SEQ ID NO: 36 or 37, but an antibody including such a VL CDR1 is still suitable for such use.

The modified CDR1 of the VL chain of the antibody or binding fragment thereof may comprise or consist of the amino acid sequence QSL-X₁-D-X₂-D-X₃-KTY, wherein X₁ is V or L, X₂ is T, S, A or N and X₃ is G or A, provided that the amino acid sequence is not QSLLDSDGKTY (SEQ ID NO: 36) or QSLVDSDGKTY (SEQ ID NO: 37). The modified CDR1 of the VL chain of the antibody or binding fragment thereof shows reduced isomerisation, in comparison with the unmodified CDR1 of SEQ ID NO: 36 or 37, but maintains the binding properties of the unmodified CDR1.

The amino acid sequences of the CDRs for the VH of a particular antibody or binding fragment thereof are shown in SEQ ID NOs: 1, 2 and 3. The CDRs 2 and 3 for the VL are shown in SEQ ID NOs: 4 and 5.

The amino acid sequences of the VH and VL of a particular antibody or binding fragment thereof are given in SEQ ID NOs: 11 and 13. The CDRs for the VH are shown in SEQ ID NOs: 1, 2 and 3. The CDRs for the VL are shown in SEQ ID NOs: 6, 4 and 5.

The amino acid sequences of the VH and VL of another antibody or binding fragment thereof are given in SEQ ID NOs: 11 and 14. The CDRs for the VH are shown in SEQ ID NOs: 1, 2 and 3. The CDRs for the VL are shown in SEQ ID NOs: 7, 4 and 5.

The amino acid sequences of the VH and VL of another antibody or binding fragment thereof are given in SEQ ID NOs: 11 and 15. The CDRs for the VH are shown in SEQ ID NOs: 1, 2 and 3. The CDRs for the VL are shown in SEQ ID NOs: 8, 4 and 5.

The amino acid sequences of the VH and VL of another antibody or binding fragment thereof are given in SEQ ID NOs: 11 and 16. The CDRs for the VH are shown in SEQ ID NOs: 1, 2 and 3. The CDRs for the VL are shown in SEQ ID NOs: 9, 4 and 5.

The amino acid sequences of the VH and VL of another antibody or binding fragment thereof are given in SEQ ID NOs: 11 and 17. The CDRs for the VH are shown in SEQ ID NOs: 1, 2 and 3. The CDRs for the VL are shown in SEQ ID NOs: 10, 4 and 5.

The amino acid sequences of the VH and VL of another antibody or binding fragment thereof are given in SEQ ID NOs: 12 and 13. The CDRs for the VH are shown in SEQ ID NOs: 1, 2 and 3. The CDRs for the VL are shown in SEQ ID NOs: 6, 4 and 5.

The amino acid sequences of the VH and VL of another antibody or binding fragment thereof are given in SEQ ID NOs: 12 and 14. The CDRs for the VH are shown in SEQ ID NOs: 1, 2 and 3. The CDRs for the VL are shown in SEQ ID NOs: 7, 4 and 5.

The amino acid sequences of the VH and VL of another antibody or binding fragment thereof are given in SEQ ID NOs: 12 and 15. The CDRs for the VH are shown in SEQ ID NOs: 1, 2 and 3. The CDRs for the VL chain are shown in SEQ ID NOs:8,4 and 5.

The amino acid sequences of the VH and VL of another antibody or binding fragment thereof are given in SEQ ID NOs: 12 and 16. The CDRs for the VH are shown in SEQ ID NOs: 1, 2 and 3. The CDRs for the VL are shown in SEQ ID NOs: 9, 4 and 5.

The amino acid sequences of the VH and VL of another antibody or binding fragment thereof are given in SEQ ID NOs: 12 and 17. The CDRs for the VH are shown in SEQ ID NOs: 1, 2 and 3. The CDRs for the VL are shown in SEQ ID NOs: 10, 4 and 5.

The antibody may comprise the heavy chain variable domain amino acid sequence of SEQ ID NO: 11, the light chain variable domain amino acid sequence of SEQ ID NO: 16, a heavy chain constant region amino acid sequence comprising SEQ ID NO: 23 or 56, and the light chain constant region amino acid sequence of SEQ ID NO: 24.

The antibody may comprise the heavy chain variable domain amino acid sequence of SEQ ID NO: 11, the light chain variable domain amino acid sequence of SEQ ID NO: 16, the heavy chain constant region amino acid sequence of SEQ ID NO: 23 or 56, and the light chain constant region amino acid sequence of SEQ ID NO: 24.

An antibody or binding fragment thereof may comprise one or more of the CDR sequences of any one of the specific antibodies as described above, except that the CDR1 of the VL is always present as either comprising or consisting of the amino acid sequence QSL-X₁-D-X₂-D-X₃-KTY, wherein X₁ is V or L, X₂ is T, S, A or N and X₃ is G or A, provided that the amino acid sequence is not QSLLDSDGKTY (SEQ ID NO: 36) or QSLVDSDGKTY (SEQ ID NO: 37), or either comprises or consists of SEQ ID NOs: 6, 7, 8, 9 or 10.

An antibody or binding fragment thereof may comprise one or more VH CDR sequences and alternatively or additionally one or more VL CDR sequences of said specific antibody, in addition to VL CDR1. An antibody or binding fragment thereof may comprise one, two or all three of the VH CDR sequences of a specific antibody or binding fragment thereof as described above and alternatively or additionally one, two or all three of the VL chain CDR sequences of said specific antibody or binding fragment thereof, including VL CDR1. An antibody or binding fragment thereof may comprise all six CDR sequences of a specific antibody or binding fragment as described above. By way of example, an antibody may comprise one of SEQ ID NO: 6, 7, 8, 9 or 10 and one or more of SEQ ID NOs: 1, 2, 3, 4 and 5.

The modified CDR1 of the VL chain of the antibody or binding fragment thereof may comprise or consist of the amino acid sequence QSL-Z₁-Z₂-Z₃-Z₄-Z₅-KTY, wherein Z₁ is V or L, Z₂ is D or E, Z₃ is T, S, A or N, Z₄ is D, E, S or A and Z₅ is G or A, provided that the amino acid sequence is not QSLLDSDGKTY (SEQ ID NO: 36) or QSLVDSDGKTY (SEQ ID NO: 37). The modified CDR1 of the VL chain of the antibody or binding fragment thereof of the invention shows reduced isomerisation, in comparison with the unmodified CDR1 of SEQ ID NO: 36 or 37, but maintains the binding properties of the unmodified CDR1. The modified CDR1 of the VL chain of the antibody or binding fragment thereof of the invention may comprise or consist of SEQ ID NO: 6, 7, 8, 9, 10, 41, 42, 43, 44, 45, 46, 47, 48, 49, 50, 51 or 52. The antibody may comprise one of SEQ ID NO: 6, 7, 8, 9, 10, 41, 42, 43, 44, 45, 46, 47, 48, 49, 50, 51 or 52, and one or more of SEQ ID NOs: 1, 2, 3, 4 and 5. The antibody may comprise one of SEQ ID NO: 6, 7, 8, 9, 10, 41, 42, 43, 44, 45, 46, 47, 48, 49, 50, 51 or 52, and all of SEQ ID NOs: 1, 2, 3, 4 and 5.

An antibody or binding fragment thereof suitable for use in the methods of the invention may alternatively comprise a variant of one of these heavy chain variable domains or CDR sequences in CDR2 or 3 of the VL. For example, a variant may be a substitution, deletion or addition variant of any of the above amino acid sequences.

A variant antibody may comprise 1, 2, 3, 4, 5, up to 10, up to 20, up to 30 or more amino acid substitutions and/or deletions from the specific sequences and fragments discussed above, whilst maintaining the activity of the antibodies described herein. "Deletion" variants may comprise the deletion of, for example, 1, 2, 3, 4 or 5 individual amino acids or of one or more small groups of amino acids such as 2, 3, 4 or 5 amino acids. "Small groups of amino acids" can be defined as being sequential, or in close proximity but not sequential, to each other. "Substitution" variants preferably involve the replacement of one or more amino acids with the same number of amino acids and making conservative amino acid substitutions. For example, an amino acid may be substituted with an alternative amino acid having similar properties, for example, another basic amino acid, another acidic amino acid, another neutral amino acid, another charged amino acid, another hydrophilic amino acid, another hydrophobic amino acid, another polar amino acid, another aromatic amino acid, another aliphatic amino acid, another tiny amino acid, another small amino acid or another large amino acid. Some properties of the 20 main amino acids, which can be used to select suitable substituents, are as follows:

| | | | |
|---|---|---|---|
| Ala | aliphatic, hydrophobic, neutral | Met | hydrophobic, neutral |
| Cys | polar, hydrophobic, neutral | Asn | polar, hydrophilic, neutral |
| Asp | polar, hydrophilic, charged (-) | Pro | hydrophobic, neutral |
| Glu | polar, hydrophilic, charged (-) | Gln | polar, hydrophilic, neutral |
| Phe | aromatic, hydrophobic, neutral | Arg | polar, hydrophilic, charged (+) |
| Gly | aliphatic, neutral | Ser | polar, hydrophilic, neutral |
| His | aromatic, polar, hydrophilic, charged (+) | Thr | polar, hydrophilic, neutral |
| Ile | aliphatic, hydrophobic, neutral | Val | aliphatic, hydrophobic, neutral |
| Lys | polar, hydrophilic, charged (+) | Trp | aromatic, hydrophobic, neutral |
| Leu | aliphatic, hydrophobic, neutral | Tyr | aromatic, polar, hydrophobic |

Preferred "derivatives" or "variants" include those in which instead of the naturally occurring amino acid the amino acid, which appears in the sequence, is a structural analog thereof. Amino acids used in the sequences may also be derivatized or modified, e.g. labelled, providing the function of the antibody is not significantly adversely affected.

Derivatives and variants as described above may be prepared during synthesis of the antibody or by post-production modification, or when the antibody is in recombinant form using the known techniques of site-directed mutagenesis, random mutagenesis, or enzymatic cleavage and/or ligation of nucleic acids.

Preferably variant antibodies have an amino acid sequence which has more than 60%, or more than 70%, e.g. 75 or 80%, preferably more than 85%, e.g. more than 90%, 95%, 96%, 97%, 98% or 99% amino acid identity to the VL and/or VH, or a fragment thereof, of an antibody disclosed herein. This level of amino acid identity may be seen across the full-length of the relevant SEQ ID NO sequence or over a part of the sequence, such as across 20, 30, 50, 75, 100, 150, 200 or more amino acids, depending on the size of the full-length polypeptide.

Preferably the variant antibodies comprise one or more of the CDR sequences as described herein.

In connection with amino acid sequences, "sequence identity" refers to sequences, which have the stated value when assessed using ClustalW (Thompson JD et al., 1994, Nucleic Acid Res., 22, 4673-4680) with the following parameters:
Pairwise alignment parameters -Method: slow/accurate, Matrix: PAM, Gap open penalty: 10.00, Gap extension penalty: 0.10;
Multiple alignment parameters -Matrix: PAM, Gap open penalty: 10.00, % identity for delay: 30, Penalize end gaps: on, Gap separation distance: 0, Negative matrix: no, Gap extension penalty: 0.20, Residue-specific gap penalties: on, Hydrophilic gap penalties: on, Hydrophilic residues: G, P, S, N, D, Q, E, K, R. Sequence identity at a particular residue is intended to include identical residues, which have simply been derivatized.

The methods of the present invention may use antibodies having specific VH and VL amino acid sequences and variants and fragments thereof, which maintain the function or activity of these VHs and VLs.

Accordingly, the methods of the present invention may use antibodies or binding fragments thereof comprising variants of the VH that retain the ability of specifically binding a citrullinated epitope on human deiminated human histone 2A and/or histone 4. A variant of the heavy chain may have at least 70%, at least 80%, at least 85%, at least 90%, at least 95%, at least 96%, at least 97%, at least 98% or at least 99% amino acid sequence identity to the unmodified VH. The variant of the VH may comprise a fragment of at least 7 amino acids of hVH22. 101f or hVH22.101HC9 (SEQ ID NO: 11 and 12, respectively), wherein the antibody or binding fragment thereof retains the ability of being specifically reactive with a citrullinated epitope on deiminated human histone 2A and/or histone 4; or a variant of hVH22.101f or hVH22.101HC9 (SEQ ID NO: 11 and 12, respectively) having at least 70% amino acid sequence identity to a sequence of hVH22.101f or hVH22.101HC9 (SEQ ID NO: 11 and 12, respectively), wherein the antibody or binding fragment thereof retains the ability of being specifically reactive with a citrullinated epitope on deiminated human histone 2A and/or histone 4.

Representative antibodies that bind to citrullinated epitopes on deiminated human histone 2A and histone 4 are described in for example, WO2009147201, WO2011070172, WO2016092082, and WO2020038963. Each of these documents, and the antibodies disclosed therein (including all CDR sequences, variable region sequences and constant region sequences of both the heavy and light chains), is herein incorporated by reference. In particular, the antibodies designated RhmAb2.102, RhmAb2.108, RhmAb2.109, RhmAb2.110, RhmAb2.111 RhmAb2.112, MQ22.101, MQ22.102 and MQ22.101b/d in WO2016092082, and any antigen binding fragment thereof, are each incorporated by reference. Similarly, the antibodies disclosed with identifiers of the format hMQ22.101x/y in WO2020038963, and any antigen binding fragment thereof, are each incorporated by reference. The antibodies disclosed in WO2020038963 are particularly preferred and are discussed in more detail below. The antibody referred to in WO2020038963 as hMQ22.101f/LC41 is most preferred. This antibody may be described herein as CIT-013.

### Polynucleotides, vectors and host cells

The present invention also encompasses polynucleotides, vectors and expression vectors encoding the antibody or binding fragments thereof described herein.

The invention also relates to polynucleotides that encode any antibody or fragment as described herein. The terms "nucleic acid molecule" and "polynucleotide" are used interchangeably herein and refer to a polymeric form of nucleotides of any length, either deoxyribonucleotides or ribonucleotides, or analogs thereof. Non-limiting examples of polynucleotides include a gene, a gene fragment, messenger RNA (mRNA), cDNA, genomic DNA, recombinant polynucleotides, plasmids, vectors, isolated DNA of any sequence, isolated RNA of any sequence, nucleic acid probes, and primers. A polynucleotide may be provided in isolated or purified form.

A nucleic acid sequence which "encodes" a selected polypeptide is a nucleic acid molecule, which is transcribed (in the case of DNA) and translated (in the case of mRNA) into a polypeptide *in vivo* when placed under the control of appropriate regulatory sequences. The boundaries of the coding sequence are determined by a start codon at the 5' (amino) terminus and a translation stop codon at the 3' (carboxy) terminus. For the purposes of this disclosure, such nucleic acid sequences can include, but are not limited to, cDNA from viral, prokaryotic or eukaryotic mRNA, genomic sequences from viral or prokaryotic DNA or RNA, and even synthetic DNA sequences. A transcription termination sequence may be located 3' to the coding sequence. In one embodiment, a polynucleotide comprises a sequence, which encodes a VH or VL amino acid sequence as described above. The polynucleotide may encode the VH or VL sequence of a specific antibody or binding fragment thereof as disclosed herein.

An antibody or binding fragment thereof may thus be produced from or delivered in the form of a polynucleotide, which encodes, and is capable of expressing it. Where the antibody comprises two or more chains, a polynucleotide may encode one or more antibody chains. For example, a polynucleotide may encode an antibody light chain, an antibody heavy chain or both. Two polynucleotides may be provided, one of which encodes an antibody light chain and the other of which encodes the corresponding antibody heavy chain. Such a polynucleotide or pair of polynucleotides may be expressed together such that an antibody is generated.

Polynucleotides can be synthesised according to methods well known in the art, as described by way of example in Sambrook J et al. (1989, Molecular cloning: a laboratory manual; Cold Spring Harbor: New York: Cold Spring Harbor Laboratory Press).

The nucleic acid molecules of the present invention may be provided in the form of an expression cassette, which includes control sequences operably linked to the inserted sequence, thus allowing for expression of the antibody of the invention *in vivo.* These expression cassettes, in turn, are typically provided within vectors (e.g., plasmids or recombinant viral vectors). Such an expression cassette may be administered directly to a host subject. Alternatively, a vector comprising a polynucleotide may be administered to a host subject. Preferably the polynucleotide is prepared and/or administered using a genetic vector. A suitable vector may be any vector, which is capable of carrying a sufficient amount of genetic information, and allowing expression of a polypeptide, such as the antibody or binding fragment thereof defined above.

Also disclosed are expression vectors that comprise such polynucleotide sequences. Such expression vectors are routinely constructed in the art of molecular biology and may for example involve the use of plasmid DNA and appropriate initiators, promoters, enhancers and other elements, such as for example polyadenylation signals, which may be necessary, and which are positioned in the correct orientation, in order to allow for expression of a peptide of the invention. Other suitable vectors would be apparent to persons skilled in the art. By way of further example in this regard we refer to Sambrook J et al. (1989, Molecular cloning: a laboratory manual; Cold Spring Harbor: New York: Cold Spring Harbor Laboratory Press).

A person skilled in the art may use the sequences described herein to clone or generate cDNA or genomic sequences for instance such as described in the below examples. Cloning of these sequences in an appropriate eukaryotic expression vector, like pcDNA3 (Invitrogen), or derivates thereof, and subsequent transfection of mammalian cells (like CHO cells) with combinations of the appropriate light and heavy chain-containing vectors will result in the expression and secretion of the antibodies described herein.

The skilled person may also make analogues of the antibodies or binding fragments thereof as described herein by using the specific binding domains of the antibody sequences and express them in a different context, such as a polypeptide, such as a fusion protein. This is well known in the art.

Also disclosed are cells that have been modified to express an antibody. Such cells include transient, or preferably stable higher eukaryotic cell lines, such as mammalian cells or insect cells, lower eukaryotic cells, such as yeast or prokaryotic cells, such as bacterial cells. Particular examples of cells, which may be modified by insertion of vectors or expression cassettes encoding for an antibody of the invention, include mammalian HEK293, CHO, HeLa, NS0 and COS cells. Preferably the cell line selected will be one which is not only stable, but also allows for mature glycosylation.

Such cell lines may be cultured using routine methods to produce an antibody or binding fragment thereof, or may be used therapeutically or prophylactically to deliver antibodies or binding fragments thereof to a subject. Alternatively, polynucleotides, expression cassettes or vectors of the invention may be administered to a cell from a subject *ex vivo* and the cell then returned to the body of the subject.

Disclosed herein is a process for the production of an antibody or binding fragment thereof that specifically binds to a citrullinated epitope on deiminated human histone 2A and/or histone 4, comprising culturing a host cell as described herein and isolating the antibody or binding fragment thereof from said cell.

### Pharmaceutical compositions

When used in the methods of the invention, the antibody or binding fragment as defined above may be provided as a pharmaceutical composition comprising the antibody or binding fragment thereof. The invention therefore encompasses pharmaceutical compositions comprising the antibodies or binding fragments thereof and a pharmaceutically acceptable carrier, for use in the methods of the invention.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for parenteral, e.g. intravenous, intraocular, intramuscular, subcutaneous, intradermal or intraperitoneal administration (e.g. by injection or infusion). In certain embodiments, a pharmaceutically acceptable carrier comprises at least one carrier selected from the group consisting of a co-solvent solution, liposomes, micelles, liquid crystals, nanocrystals, nanoparticles, emulsions, microparticles, microspheres, nanospheres, nanocapsules, polymers or polymeric carriers, surfactants, suspending agents, complexing agents such as cyclodextrins or adsorbing molecules such as albumin, surface active particles, and chelating agents. In further embodiments, a polysaccharide comprises hyaluronic acid and derivatives thereof, dextran and derivatives thereof, cellulose and derivatives thereof (e.g. methylcellulose, hydroxy-propylcellulose, hydroxypropylmethylcellulose, carboxymethylcellulose, cellulose acetate phthalate, cellulose acetate succinate, cellulose acetate butyrate, hydroxypropylmethyl-cellulose phthalate), chitosan and derivative thereof, [beta]-glucan, arabinoxylans, carrageenans, pectin, glycogen, fucoidan, chondrotin, dermatan, heparan, heparin, pentosan, keratan, alginate, cyclodextrins, and salts and derivatives, including esters and sulfates, thereof.

Preferred pharmaceutically acceptable carriers comprise aqueous carriers or diluents. Examples of suitable aqueous carriers that may be employed in the pharmaceutical compositions of the invention include water, buffered water and saline. Examples of other carriers include ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants. In many cases, it will be preferable to include isotonic agents, for example, sugars, polyalcohols, such as mannitol, sorbitol, or sodium chloride in the composition.

A pharmaceutical composition may include a pharmaceutically acceptable antioxidant. These compositions may also contain adjuvants, such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents, which delay absorption such as aluminium monostearate and gelatin.

Therapeutic compositions typically must be sterile and stable under the conditions of manufacture and storage. The pharmaceutical composition can be formulated as a solution, microemulsion, liposome, or other ordered structure suitable to high drug concentration.

Sterile injectable solutions can be prepared by incorporating the active agent (e.g. antibody) in the required amount in an appropriate solvent with one or a combination of ingredients enumerated above, as required, followed by sterilization microfiltration. Generally, dispersions are prepared by incorporating the active agent into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active agent plus any additional desired ingredient from a previously sterile-filtered solution thereof.

Pharmaceutical compositions may comprise additional active ingredients as well as an antibody as defined above. As mentioned above, compositions of the invention may comprise one or more antibodies. They may also comprise additional therapeutic or prophylactic active agents.

Depending on the route of administration, the antibody or binding fragment thereof may be coated in a material to protect the antibody from the action of acids and other natural conditions that may inactivate or denature the antibody.

In a preferred embodiment, the pharmaceutical composition according to the invention is in a form selected from the group consisting of an aqueous solution, a gel, a hydrogel, a film, a paste, a cream, a spray, an ointment, or a wrap.

In further embodiments, the pharmaceutical compositions described herein can be administered by a route such as intravenous, subcutaneous, intraocular, intramuscular, intra-articular, intradermal, intraperitoneal, spinal or by other parenteral routes of administration, for example by injection or infusion. Administration may be rectal, oral, ocular, topical, epidermal or by the mucosal route. Administration may be local, including by inhalation. In a preferred embodiment, the pharmaceutical composition is administered intravenously or subcutaneously.

Also disclosed herein are kits comprising antibodies or other compositions of the invention and instructions for use. The kit may further contain one or more additional reagents, such as an additional therapeutic or prophylactic agent as discussed herein.

### Methods for the prevention and treatment of disease

The present invention provides a method of inhibiting the formation of eosinophil extracellular traps (EETs), the method comprising administering an antibody or binding fragment thereof that specifically binds to a citrullinated epitope on deiminated human histone 2A and/or histone 4 to a sample or a subject in which eosinophils are present. The method may be for the prevention or treatment of a disease or condition in a subject, in which case the method comprises administering said antibody or binding fragment thereof to the subject in a prophylactically or therapeutically effective amount. Also provided is an antibody or binding fragment thereof that specifically binds to a citrullinated epitope on deiminated human histone 2A and/or histone 4 for use in the said method for the prevention or treatment of a disease or condition in a subject. Also provided is an antibody or binding fragment thereof that specifically binds to a citrullinated epitope on deiminated human histone 2A and/or histone 4 for use in the manufacture of a medicament for use in the said method for the prevention or treatment of a disease or condition in a subject.

In therapeutic applications, antibodies or compositions are administered to a subject already suffering from a disorder or condition, in an amount sufficient to cure, alleviate or partially arrest the condition or one or more of its symptoms. Such therapeutic treatment may result in a decrease in severity of disease symptoms, or an increase in frequency or duration of symptom-free periods. An amount adequate to accomplish this is defined as "therapeutically effective amount". Effective amounts for a given purpose will depend on the severity of the disease or injury as well as the weight and general state of the subject. In prophylactic applications, polypeptides or compositions are administered to a subject not yet exhibiting symptoms of a disorder or condition, in an amount sufficient to prevent or delay the development of symptoms. Such an amount is defined as a "prophylactically effective amount". As used herein, the term "subject" includes any vertebrate, typically any mammal, such as human or horse. The subject is preferably human.

In particular embodiments, the antibody or binding fragment thereof may be linked (directly or indirectly) to another moiety. The other moiety may be a therapeutic agent such as a drug. The other moiety may be a detectable label. The other moiety may be a binding moiety, such as an antibody or a polypeptide binding domain specific for a therapeutic target. The antibody or binding fragment thereof of the invention may be a bispecific antibody.

The therapeutic agent or a detectable label may be directly attached, for example by chemical conjugation, to an antibody or binding fragment thereof of the invention. Methods of conjugating agents or labels to an antibody are known in the art. For example, carbodiimide conjugation (Bauminger S and Wilchek M, 1980, Methods Enzymol., 70, 151-159) may be used to conjugate a variety of agents, including doxorubicin, to antibodies or peptides. The water-soluble carbodiimide, 1-ethyl-3-(3-dimethylaminopropyl) carbodiimide (EDC) is particularly useful for conjugating a functional moiety to a binding moiety.

Other methods for conjugating a moiety to antibodies can also be used. For example, sodium periodate oxidation followed by reductive alkylation of appropriate reactants can be used, as can glutaraldehyde cross-linking. However, it is recognised that, regardless of which method of producing a conjugate of the invention is selected, a determination must be made that the antibody maintains its targeting ability and that the functional moiety maintains its relevant function.

The therapeutic agent linked to the antibody may comprise a polypeptide or a polynucleotide encoding a polypeptide which is of therapeutic benefit. Examples of such polypeptides include anti-proliferative or anti-inflammatory cytokines.

The antibody may be linked to a detectable label. By "detectable label" it is meant that the antibody is linked to a moiety which, when located at the target site following administration of the antibody into a patient, may be detected, typically non-invasively from outside the body and the site of the target located. Thus, the antibody may be useful in imaging and diagnosis.

Typically, the label is or comprises a radioactive atom which is useful in imaging. Suitable radioactive atoms include 99mTc and 123I for scintigraphic studies. Other labels include, for example, spin labels for magnetic resonance imaging (MRI) such as 1231 again, 1311, 111In, 19F, 13C, 15N, 170, gadolinium, manganese or iron. Clearly, the sufficient amount of the appropriate atomic isotopes must be linked to the antibody in order for the molecule to be readily detectable.

The radio- or other lafbels may be incorporated in known ways. For example, the antibody, or fragment thereof, may be biosynthesised or may be synthesised by chemical amino acid synthesis using suitable amino acid precursors involving, for example, fluorine-19 in place of hydrogen. Labels such as 99mTc, 1231, 186Rh, 188Rh and 111In can, for example, be attached via cysteine residues in polypeptides. Yttrium-90 can be attached via a lysine residue. Preferably, the detectable label comprises a radioactive atom, such as, for example technetium-99m or iodine-123. Alternatively, the detectable label may be selected from the group comprising: iodine-123; iodine-131; indium-Ill; fluorine-19; carbon-13; nitrogen-15; oxygen-17; gadolinium; manganese; iron.

In one embodiment, an antibody of the invention is able to bind selectively to a directly or indirectly cytotoxic moiety or to a detectable label. Thus, in this embodiment, the antibody is linked to a moiety which selectively binds to a further compound or component which is cytotoxic or readily detectable.

An antibody or binding fragment, or a composition comprising said antibody or fragment, may be administered via one or more routes of administration using one or more of a variety of methods known in the art. As will be appreciated by the skilled artisan, the route and/or mode of administration will vary depending upon the desired results. Preferred routes of administration for antibodies or compositions of the invention include intravenous, subcutaneous, intraocular, intramuscular, intradermal, intraperitoneal, spinal or other parenteral routes of administration, for example by injection or infusion. The phrase "parenteral administration" as used herein means modes of administration other than enteral and topical administration, usually by injection. Administration may be rectal, oral, ocular, topical, epidermal or by the mucosal route. Administration may be local, including peritumoral, juxtatumoral, intratumoral, to the resection margin of tumors, intralesional, perilesional, by intra cavity infusion, intravesicle administration, or by inhalation. In a preferred embodiment, the pharmaceutical composition is administered intravenously or subcutaneously.

A suitable dosage of an antibody or binding fragment thereof may be determined by a skilled medical practitioner. Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular antibody employed, the route of administration, the time of administration, the rate of excretion of the antibody, the duration of the treatment, other drugs, compounds and/or materials used in combination with the particular compositions employed, the age, sex, weight, condition, general health and prior medical history of the patient being treated, and like factors well known in the medical arts.

A suitable dose of an antibody or binding fragment thereof may be, for example, in the range of from about 0.1 µg/kg to about 100 mg/kg body weight of the patient to be treated. For example, a suitable dosage may be from about 1 µg/kg to about 50 mg/kg body weight per week, from about 100 µg/kg to about 25 mg/kg body weight per week or from about 10 µg/kg to about 12.5 mg/kg body weight per week.

A suitable dosage may be from about 1 µg/kg to about 50 mg/kg body weight per day, from about 100 µg/kg to about 25 mg/kg body weight per day or from about 10 µg/kg to about 12.5 mg/kg body weight per day.

Dosage regimens may be adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Antibodies may be administered in a single dose or in multiple doses. The multiple doses may be administered via the same or different routes and to the same or different locations. Alternatively, antibodies can be administered as a sustained release formulation, in which case less frequent administration is required. Dosage and frequency may vary depending on the half-life of the antibody in the patient and the duration of treatment that is desired. The dosage and frequency of administration can also vary depending on whether the treatment is prophylactic or therapeutic. In prophylactic applications, a relatively low dosage may be administered at relatively infrequent intervals over a long period of time. In therapeutic applications, a relatively high dosage may be administered, for example until the patient shows partial or complete amelioration of symptoms of disease.

Combined administration of two or more agents may be achieved in a number of different ways. In one embodiment, the antibody or binding fragment thereof and the other agent may be administered together in a single composition. In another embodiment, the antibody and the other agent may be administered in separate compositions as part of a combined therapy. For example, the antibody or binding fragment thereof may be administered before, after or concurrently with the other agent.

### Diseases to be diagnosed, treated or prevented

The methods disclosed herein may be for the diagnosis, treatment or prevention of any disease or condition which includes an EET-associated pathology. An EET-associated pathology typically means a pathology which is mediated in whole or in part by the formation of EETs. Such a pathology is typically present in any disease or condition which is mediated in whole or in part, or preferably which is mediated primarily, by eosinophils. Such diseases or conditions may be described herein as eosinophilic or eosinophil-associated. Therefore, put another way, the methods disclosed herein may be for the diagnosis, treatment or prevention of an eosinophilic disease or condition.

An eosinophilic disease or condition may be defined as a disease or condition in which eosinophils are present in elevated numbers in the tissue or organ affected, relative to the same tissue or organ in a healthy individual. Eosinophilic diseases and conditions may include: an eosinophilic disease or condition of the skin; a respiratory eosinophilic disease or condition; a gastro-intestinal eosinophilic disease or condition; an allergic disease or condition; or a helminth, fungal, viral, or bacterial infection.

Eosinphilic diseases or conditions of the skin include Bullous Pemphigoid (PB), Atopic dermatitis (AD) and Chronic spontaneous Urticaria (CSU), allergic contact dermatitis, and eosinophilic cellulitis (also called Well's syndrome).

Respiratory eosinophilic diseases or conditions include Eosinophilic Asthma, Nasal Polyps, Chronic RhinoSinusitis with Nasal Polyposis (CRSwNP), Allergic sinusitis, Allergic rhinisitis, Allergic bronchopulmonary aspergillosis (a fungal infection), Eosinophilic chronic rhinosinusitis, Tropical pulmonary eosinophilia (typically a respiratory Helminth infection).

Gastro-intestinal eosinophilic diseases or conditions include Eosinophilic Esophagitis (EoE), Eosinophilic gastritis (stomach - EG), Eosinophilic gastroenteritis (stomach and small intestine - EGE), Eosinophilic enteritis (small intestine), Eosinophilic colitis (large intestine - EC), and a gastro-intestinal helminth infection such as Ascariasis or Trichinosis.

Other eosinophilic diseases or conditions include HyperEosinophilic Syndrome (HES - affects blood and various organs), Eosinophilic Granulomatosis with PolyAngitis (EGPA - affects various organs including blood vessels) and Eosinophilic otitis media (EOM - affects the middle ear), and Drug Reaction with Eosinophilic & Systemic Symptoms (DRESS - affects various organs).

The methods disclosed herein may be for the diagnosis, treatment or prevention of any of the above-listed eosinophilic diseases or conditions. Particularly preferred eosinophilic diseases or conditions include those in which the presence of EETs has been directly confirmed. Such disease and conditions include but are not limited to: Bullous Pemphigoid, Atopic dermatitis, allergic contact dermatitis, Eosinophilic Asthma, Chronic RhinoSinusitis with Nasal Polyposis (CRSwNP), Allergic sinusitis, Allergic bronchopulmonary aspergillosis, Eosinophilic chronic rhinosinusitis, Eosinophilic Esophagitis (EoE), HyperEosinophilic Syndrome (HES), Eosinophilic Granulomatosis with PolyAngitis (EGPA), Eosinophilic otitis media (EOM), and Drug Reaction with Eosinophilic & Systemic Symptoms (DRESS).

The most preferred eosinophilic diseases or conditions are those in which a correlation between EETs and disease incidence and/or severity has been directly observed. Such disease and conditions include but are not limited to: Eosinophilic Asthma, Chronic RhinoSinusitis with Nasal Polyposis (CRSwNP), Eosinophilic chronic rhinosinusitis, and Eosinophilic otitis media (EOM).

The presence of EETs and/or a role for eosinophils in diseases such as those discussed above is well-established in the art. See for example: Williams, T. L et al. (2020). "NETs and EETs, a Whole Web of Mess". Microorganisms, 8(12), 1925 and Mukherjee, M., et al. (2018). Eosinophil Extracellular Traps and Inflammatory Pathologies-Untangling the Web!. Frontiers in immunology, 9, 2763. The invention is suitable for the treatment, prevention or diagnosis of any disease recited in these documents, which are incorporated by reference.

The methods disclosed herein may also be for the diagnosis, treatment or prevention of an EET-associated pathology in a disease or condition which is only partly mediated by eosinophils. For example, diseases such as Chronic Obstructive Pulmonary Disease (COPD), Crohn's disease, ulcerative colitis, dermatitis herpetiformis, thrombosis, and atherosclerosis may exhibit multiple pathologies caused by multiple cell types, and so may not be defined as "eosinophilic". However, they may nonetheless exhibit EET-associated pathology and thus be diagnosed, treated or prevented by the methods disclosed herein.

### Alternative methods

The present invention provides a method of inhibiting or detecting the formation of eosinophil extracellular traps (EETs), the method comprising administering an antibody or binding fragment thereof that specifically binds to a citrullinated epitope on deiminated human histone 2A and/or histone 4 to a sample or a subject in which eosinophils are present. The method may be for the *ex vivo* inhibition or detection of EET formation in a sample, in which case the method comprises administering said antibody or binding fragment thereof to the sample and incubating under conditions suitable for binding to occur. In other words, the conditions permit formation of an antibody-target complex. The method may optionally include determining whether said complex has formed.

In such methods, a sample is contacted with a suitable antibody or fragment under conditions suitable for binding to occur. Suitable conditions include incubation for at least 1 minute, 2 minutes, 3 minutes, 4 minutes, 5 minutes, 6 minutes, 7 minutes, 8 minutes, 9 minutes, 10 minutes, or longer. Incubation preferably takes place at room temperature, more preferably at approximately 20°C, 25°C, 30°C, 35°C, 40°C or 45°C, and most preferably at approximately 37°C. The methods described above may be carried out under any suitable pH, but typically at around pH 6.5. to 7.5. The method may be conducted in any suitable buffer, such as tris buffered saline (TBS) or phosphate buffered saline (PBS).

The detection or analysis of the sample to determine whether binding has taken place may be assessed by any suitable analytical method, such as but not limited to mass spectrometry, HPLC, affinity chromatography, gel electrophoresis, SDS-PAGE, ELISA, lectin blotting, spectrometry, capillary electrophoresis, flow cytometry, microscopy and other standard laboratory techniques for analysis.

The antibody or binding fragment thereof may be bound to a solid support or may be labeled or conjugated to another chemical group or molecule as described above, to assist with detection. For example, typical chemical groups include fluorescent labels such as Fluorescein isothiocyanate (FITC) or Phycoerythrin (PE), or tags such as biotin.

The sample is typically a sample of a body fluid obtained from a subject, such as serum or blood. The method may comprise processing the sample before administering the antibody or fragment, for example by to isolate the eosinophils. The sample may be a sample taken from a subject, preferably a human subject in whom the presence of EET-associated pathology may be confirmed or suspected. The results obtained may be used for a diagnostic purpose, for example to detect or confirm the presence of EET-associated pathology in the subject, including for example in any of the diseases recited in the previous section. Such a use may involve comparison of the results obtained from the subject to those obtained using a sample obtained from a healthy control. The presence of EET-associated pathology in the subject may be confirmed or suspected due to the presence of one or more symptoms of eosinophilic disease, including any eosinophilic disease as described herein.

The present invention is further illustrated by the following examples which should not be construed as further limiting. The contents of all figures and all references, patents and published patent applications cited throughout this application are expressly incorporated herein by reference.

### Examples

### Example 1: Inhibition of extracellular DNA release from eosinophils by CIT-013 antibody upon stimulation with A23187 or PMA.

Eosinophils were isolated from 20 ml blood of healthy donors by Ficoll gradient centrifugation followed by ACK lysis of red blood cells and subsequent negative selection using the eosinophil isolation kit (Miltenyi Biotec) with magnetic beads according to manufacturer's protocol. Isolated eosinophils (∼90% purity based on CD16⁻ Siglec-8⁺ expression measured by flow cytometry) were stimulated with 2 µM A23187 or 100 nM phorbol 12-myristate 13-acetate (PMA) in absence or presence of CIT-013 or an isotype control antibody (25 µg/ml). As negative control, cells were seeded without stimulation or antibody exposure (untreated). Sytox Green, a cell impermeable dye, was present in all wells to visualize DNA. Four images were taken per well for phase contrast and Sytox Green every 30 min using Incucyte live-cell imaging and analysis system SX1 (Sartorius). a) representative image taken after 4 hours of stimulation is shown for the various conditions. The Sytox green signal is depicted in grayscale. Scale bars: 100 µm b) The data were analysed using the Incucyte SX1 software for extracellular traps. These extracellular traps were determined as Sytox Green signal with a relatively low mean intensity due to spreading of the DNA (between 18 and 80) and an area larger than the average cell area (area between 315 µm² and 4000 µm² -based on measurement of randomly chosen example images from the experiment). The number of cells at the beginning of the experiment was determined in the phase contrast image as events with an area above 70 µm² (value again based on evaluation of the specific experiment). The results are expressed as the relative number of extracellular traps compared to the number of cells at the beginning. In the graphs the mean and standard deviation of three wells per condition is depicted over time for eosinophils-derived from each donor (n=3). The graphs show clear reduction in the percentage of eosinophilic extracellular traps in the presence of CIT-013 compared to the isotype control antibody.

### Sequence listing

SEQ ID NO: 1- CDR1 of msVH22.101 and hVH22.101(HC)x
   GYTFTNYG
SEQ ID NO: 2- CDR2 of msVH22.101 and hVH22.101(HC)x
   INTYSGEA
SEQ ID NO: 3- CDR3 of msVH22.101 and hVH22.101(HC)x
   LRGYTYQSFDEGGDY
SEQ ID NO: 4- CDR2 of msVL22.101 and hVL22.101(LC)y
   LVS
SEQ ID NO: 5- CDR3 of msVL22.101 and hVL22.101(LC)y
   WQGTHFPYT
SEQ ID NO: 6- CDR1 of hVL22.101LC17
   QSLLDTDGKTY
SEQ ID NO: 7- CDR1 of hVL22.101LC21
   QSLLDSDAKTY
SEQ ID NO: 8- CDR1 of hVL22.101LC27
   QSLLDTDAKTY
SEQ ID NO: 9- CDR1 of hVL22.101LC41
   QSLLDADGKTY
SEQ ID NO: 10- CDR1 of hVL22.101LC42
   QSLLDNDGKTY
SEQ ID NO: 11- hVH22.101f 
RIQLVQSGAEVKKPGASVKVSCKASGYTFTNYGMHWVRQAPGQGLEWMGWINTYSGEATYAQKFQGRVTMTRDTSISTAYM
ELSRLRSDDTAVYYCLRGYTYQSFDEGGDYWGQGTLVTVSS
SEQ ID NO: 12- hVH22.101HC9 
RIQLVQSGAEVKKPGASVKVSCKASGYTFTNYGMHWVRQAPGQGLEWMGWINTYSGEATYVDDFQGRVTMTRDTSISTAYM
ELSRLRSDDTAVYYCLRGYTYQSFDEGGDYWGQGTLVTVSS
SEQ ID NO: 13- hVL22.101LC17 
DVVMTQSPLSLPVTLGQPASISCRSSQSLLDTDGKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 14- hVL22.101LC21 
DVVMTQSPLSLPVTLGQPASISCRSSQSLLDSDAKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 15- hVL22.101LC27 
DVVMTQSPLSLPVTLGQPASISCRSSQSLLDTDAKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 16- hVL22.101LC41 
DVVMTQSPLSLPVTLGQPASISCRSSQSLLDADGKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 17- hVL22.101LC42 
DVVMTQSPLSLPVTLGQPASISCRSSQSLLDNDGKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 18- SEQ ID NO 1 from WO2016092082 (used in Example 1/7) from histone 2A
   SGXGKQGGKARA
   Where X is citrulline
SEQ ID NO: 19- SEQ ID NO 2 from WO2016092082, (used in Example 7) from histone 4
   SGXGKGGKGLGKGGAKRHRKVLR
   Where X is citrulline
SEQ ID NO: 20- Shortened SEQ ID NO 2 from WO2016092082 (used in Example 7) from histone 4
   SGXGKGGKGLGK
   Where X is citrulline
SEQ ID NO: 21- Peptide no 4 (human histone 2A) (SEQ ID NO 24 from WO2011070172)
   QFPVGXVHRLLR
   Where X is citrulline
SEQ ID NO: 22- Peptide no 6 (human histone 2A) (SEQ ID NO 26 from WO2011070172)
   VHRLLXKGNYSE
   Where X is citrulline
SEQ ID NO: 23- Human heavy chain constant domain of IgG1 
ASTKGPSVFPLAPSSKSTSGGTAALGCLVKDYFPEPVTVSWNSGALTSGVHTFPAVLQSSGLYSLSSVVTVPSSSLGTQTY
ICNVNHKPSNTKVDKKVEPKSCDKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTISKAKGQPREPQVYTLPPSRDELTK
NQVSLTCLVKGFYPSDIAVEWESNGQPENNYKTTPPVLDSDGSFFLYSKLTVDKSRWQQGNVFSCSVMHEALHNHYTQKSL
SLSPG
SEQ ID NO: 24- Human kappa chain constant domain
SEQ ID NO: 25- msVH22.101 
RIQLVQSGPELKKPGEAVKISCKASGYTFTNYGMHWMKQTPGKDFRWMGWINTYSGEATYVDDFKGRFAFSLGTSASTAYL
QINNLKNDDTATYFCLRGYTYQSFDEGGDYWGQGTALTVSS
SEQ ID NO: 26- hVH22.101j 
QVQLVQSGAEVKKPGASVKVSCKASGYTFTNYGMHWVRQAPGQGLEWMGWINTYSGEATYAQKFQGRVTMTRDTSISTAYM
ELSRLRSDDTAVYYCLRGYTYQSFDEGGDYWGQGTLVTVSS
SEQ ID NO: 27- hVH22.101HC7 
QVQLVQSGAEVKKPGSSVKVSCKASGYTFTNYGMHWVRQAPGQGLEWMGWINTYSGEATYAQKFQGRVTITADESTSTAYM
ELSSLRSEDTAVYYCLRGYTYQSFDEGGDYWGQGTLVTVSS
SEQ ID NO: 28- hVH22.101HC8 
QVQLVQSGAEVKKPGSSVKVSCKASGYTFTNYGMHWVRQAPGQGLEWMGWINTYSGEATYVDDFQGRVTITADESTSTAYM
ELSSLRSEDTAVYYCLRGYTYQSFDEGGDYWGQGTLVTVSS
SEQ ID NO: 29- hVH22.101HC10 
QVQLVQSGAEVKKPGASVKVSCKASGYTFTNYGMHWVRQAPGQGLEWMGWINTYSGEATYVDDFQGRVTMTRDTSISTAYM
ELSRLRSDDTAVYYCLRGYTYQSFDEGGDYWGQGTLVTVSS
SEQ ID NO: 30- msVL22.101 
DVVMTQTPLTLSVTTGQPASISCKSSQSLLDSDGKTYLNWLFQRPGQSPKRLIYLVSKLDSGVPDRFTGSGSGTDFTLKIS
RVEAEDLGIYYCWQGTHFPYTFGGGTNLEIK
SEQ ID NO: 31- hVL22.101e 
DVVMTQSPLSLPVTLGQPASISCRSSQSLVDSDGKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 32- hVL22.101g 
DVVMTQSPLSLPVTLGQPASISCRSSQSLLDSDGKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 33- hVL22.101h 
DVVMTQSPLSLPVTLGQPASISCRSSQSLVASDGKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 34- hVL22.101i 
DVVMTQSPLSLPVTLGQPASISCRSSQSLVESDGKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 35- hVL22.101j 
DVVMTQSPLSLPVTLGQPASISCRSSQSLVSSDGKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 36- CDR1 of msVL22.101 and hVL22.101g
   QSLLDSDGKTY
SEQ ID NO: 37- CDR1 of hVL22.101e
   QSLVDSDGKTY
SEQ ID NO: 38- CDR1 of hVL22.101h
   QSLVASDGKTY
SEQ ID NO: 39- CDR1 of hVL22.101i
   QSLVESDGKTY
SEQ ID NO: 40- CDR1 of hVL22.101j
   QSLVSSDGKTY
SEQ ID NO: 41- CDR1 of hVL22.101LC16
   QSLLESDGKTY
SEQ ID NO: 42- CDR1 of hVL22.101LC19
   QSLLDSEGKTY
SEQ ID NO: 43- CDR1 of hVL22.101LC20
   QSLLDSSGKTY
SEQ ID NO: 44- CDR1 of hVL22.101LC22
   QSLLESEGKTY
SEQ ID NO: 45- CDR1 of hVL22.101LC23
   QSLLESSGKTY
SEQ ID NO: 46- CDR1 of hVL22.101LC24
   QSLLESDAKTY
SEQ ID NO: 47- CDR1 of hVL22.101LC25
   QSLLDTEGKTY
SEQ ID NO: 48- CDR1 of hVL22.101LC26
   QSLLDTSGKTY
SEQ ID NO: 49- CDR1 of hVL22.101LC37
   QSLLDSAGKTY
SEQ ID NO: 50- CDR1 of hVL22.101LC38
   QSLLESAGKTY
SEQ ID NO: 51- CDR1 of hVL22.101LC39
   QSLLDAEGKTY
SEQ ID NO: 52- CDR1 of hVL22.101LC40
   QSLLDNEGKTY
SEQ ID NO: 53- msFibβ XG (SEQ ID NO 37 from WO2011070172)
   EPTDSLDAXGHRPVDRR
   Where X is citrulline
SEQ ID NO: 54- msVim XS/XL (SEQ ID NO 38 from WO2011070172)
   YVTXSSAVXLXSSVP
   Where X is citrulline
SEQ ID NO: 55- Region around CDR2 of msVL22.101 and hVL22.101(LC)y
   LVSKLDS
SEQ ID NO: 56- Heavy chain constant domain of hCH22.101f

## Claims

**1. 1.** A method of inhibiting the formation of eosinophil extracellular traps (EETs), the method comprising administering an antibody or binding fragment thereof that specifically binds to a citrullinated epitope on deiminated human histone 2A and/or histone 4 to a sample or a subject in which eosinophils are present.

**2.** The method according to claim 1, which is for the prevention or treatment of a disease or condition in a subject, and which method comprises administering said antibody or binding fragment thereof to the subject in a prophylactically or therapeutically effective amount.

**3.** The method according to claim 2, wherein the disease or condition includes an EET-associated pathology.

**4.** The method according to claim 2 or 3, wherein the disease or condition is an eosinophilic disease or condition.

**5.** The method according to claim 4, wherein said eosinophilic disease or condition is an eosinophilic disease or condition of the skin; a respiratory eosinophilic disease or condition; a gastro-intestinal eosinophilic disease or condition; an allergic disease or condition; or a helminth, fungal, viral, or bacterial infection.

**6.** The method according to any one of claims 2 to 5, wherein the disease or condition is selected from: Bullous Pemphigoid, Atopic dermatitis, allergic contact dermatitis, Eosinophilic Asthma, Chronic RhinoSinusitis with Nasal Polyposis (CRSwNP), Allergic sinusitis, Allergic bronchopulmonary aspergillosis, Eosinophilic chronic rhinosinusitis, Eosinophilic Esophagitis (EoE), HyperEosinophilic Syndrome (HES), Eosinophilic Granulomatosis with PolyAngitis (EGPA), Eosinophilic otitis media (EOM), and Drug Reaction with Eosinophilic & Systemic Symptoms (DRESS).

**7.** The method according to any one of claims 2 to 6, wherein the disease or condition is selected from: Eosinophilic Asthma, Chronic RhinoSinusitis with Nasal Polyposis (CRSwNP), Eosinophilic chronic rhinosinusitis, and Eosinophilic otitis media (EOM).

**8.** The method according to claim 1, which is for the *ex vivo* inhibition or detection of EET formation in a sample, and which comprises administering said antibody or binding fragment thereof to the sample and incubating under conditions suitable for binding to occur, optionally wherein the sample is of a body fluid obtained from a subject, such as serum or blood, and optionally wherein the sample is processed for example to isolate eosinophils.

**9.** The method according to any one of the preceding claims, wherein the antibody or binding fragment thereof comprises:
a) CDR1 of VL, wherein the CDR comprises or consists of the amino acid sequence QSL-X₁-D-X₂-D-X₃-KTY, wherein X₁ is V or L, X₂ is T, S, A or N and X₃ is G or A, preferably wherein the amino acid sequence is not QSLLDSDGKTY (SEQ ID NO: 36) or QSLVDSDGKTY (SEQ ID NO: 37); and
b) at least one CDR selected from SEQ ID NOs: 1 to 5, optionally at least the CDRs of SEQ ID NO: 3 and SEQ ID NO: 5, and preferably all five CDRs of SEQ ID NOs: 1 to 5.

**9.** The method according to claim 8, wherein the antibody or binding fragment thereof comprises:
a) a VL CDR1 of SEQ ID NOs: 6, 7, 8, 9 or 10; and
b) the CDRs of SEQ ID NOs: 1 to 5;
OR
a) the VL CDRs from any one of SEQ ID NOs: 13, 14, 15, 16 or 17; and
b) the heavy chain variable domain amino acid sequence of SEQ ID NO: 11 or 12;

**10.** The method according to claim 8 or 9, wherein the antibody or binding fragment thereof comprises:
(I) a light chain variable region comprising:
a) a VL CDR1, wherein the CDR comprises or consists of the amino acid sequence QSL-X1-D-X2-D-X3-KTY, wherein X1 is V or L, X2 is T, S, A or N and X3 is G or A, preferably wherein the amino acid sequence is not QSLLDSDGKTY (SEQ ID NO: 36) or QSLVDSDGKTY (SEQ ID NO: 37), and optionally wherein said CDR1 is selected from SEQ ID NO: 6, 7, 8, 9 or 10; and
b) at least one, and preferably both, of the VL CDR2 of SEQ ID NO: 4 and the VL CDR3 of SEQ ID NO: 5;
and
(II) a heavy chain variable region comprising:
c) the amino acid sequence of SEQ ID NO: 11 or 12; or
d) a fragment of at least 7 amino acids of (c), wherein the antibody or binding fragment thereof retains the ability of being specifically reactive with a citrullinated epitope on deiminated human histone 2A and/or histone 4; or
e) a variant of (c) having at least 70% amino acid sequence identity to a sequence of (c), wherein the antibody or binding fragment thereof retains the ability of being specifically reactive with a citrullinated epitope on deiminated human histone 2A and/or hi stone 4.

**11.** The method according to any one of claims 8 to 10, wherein the antibody or binding fragment thereof comprises:
a) the heavy chain variable domain amino acid sequence of SEQ ID NO: 11 and the light chain variable domain amino acid sequence of SEQ ID NO: 13;
b) the heavy chain variable domain amino acid sequence of SEQ ID NO: 11 and the light chain variable domain amino acid sequence of SEQ ID NO: 14;
c) the heavy chain variable domain amino acid sequence of SEQ ID NO: 11 and the light chain variable domain amino acid sequence of SEQ ID NO: 15;
d) the heavy chain variable domain amino acid sequence of SEQ ID NO: 11 and the light chain variable domain amino acid sequence of SEQ ID NO: 16;
e) the heavy chain variable domain amino acid sequence of SEQ ID NO: 11 and the light chain variable domain amino acid sequence of SEQ ID NO: 17;
f) the heavy chain variable domain amino acid sequence of SEQ ID NO: 12 and the light chain variable domain amino acid sequence of SEQ ID NO: 13;
g) the heavy chain variable domain amino acid sequence of SEQ ID NO: 12 and the light chain variable domain amino acid sequence of SEQ ID NO: 14;
h) the heavy chain variable domain amino acid sequence of SEQ ID NO: 12 and the light chain variable domain amino acid sequence of SEQ ID NO: 15;
i) the heavy chain variable domain amino acid sequence of SEQ ID NO: 12 and the light chain variable domain amino acid sequence of SEQ ID NO: 16; or
j) the heavy chain variable domain amino acid sequence of SEQ ID NO: 12 and the light chain variable domain amino acid sequence of SEQ ID NO: 17.

**12.** The method according to any one of the preceding claims, wherein the antibody or binding fragment thereof specifically binds to a peptide selected from the group consisting of SEQ ID NOs: 18, 19, 20, 21 and 22, and binds deiminated human histone 2A and/or histone 4, preferably with an affinity of at least InM or less.

**13.** The method according to any one of the preceding claims, wherein (i) the antibody or binding fragment thereof is selected from the group consisting of recombinant antibodies, single chain antibodies, single chain variable fragments (scFv), variable fragments (Fv), fragment antigen-binding regions (Fab), single-domain antibodies (sdAb), VHH antibodies, nanobodies, camelids-derived single-domain antibodies, shark IgNAR-derived single-domain antibody fragments (VNAR), diabodies, triabodies, Anticalins and aptamers, and is preferably a full-length antibody, and/or (ii) wherein the antibody or binding fragment thereof is conjugated to an additional moiety.

**14.** The method according to any one of the preceding claims, wherein the antibody or binding fragment thereof comprises an Fc region, such as an IgG1, IgG2, IgG3 or IgG4 region, optionally wherein the heavy chain constant region comprises SEQ ID NO: 23 or 56, and/or the light chain constant region comprises SEQ ID NO: 24.

**15.** The method according to any one of the preceding claims, wherein the antibody comprises the heavy chain variable domain amino acid sequence of SEQ ID NO: 11, the light chain variable domain amino acid sequence of SEQ ID NO: 16, the heavy chain constant region amino acid sequence of SEQ ID NO: 23 or 56, and the light chain constant region amino acid sequence of SEQ ID NO: 24.
